# EUROPEAN PATENT SPECIFICATION

(11) **EP 0 559 428 B1**
(45) Date of publication and mention of the grant of the patent: **31.07.1996**
(21) Application number: 93301571.1
(22) Date of filing: 02.03.1993
(51) Int. Cl.: C12N 15/12, A61K 35/30, C07K 14/48

(54) **Novel polypeptides and DNAs encoding them**
Neue Polypeptide, deren kodierende DNS
Nouveaux polypeptides et ADNs codant pour ceux-ci

(30) Priority: 03.03.1992 JP 81557/92; 22.04.1992 JP 129558/92
(43) Date of publication of application: 08.09.1993
(73) Proprietor: ONO PHARMACEUTICAL CO., LTD., Chuo-ku Osaka 541 (JP)
(72) Inventor: Naitoh, Takayuki, c/o Manase Research Institute, Shimamoto-cho, Mishima-gun, Osaka (JP); Konishi, Mikio, c/o Minase Research Institute, Shimamoto-cho, Mishima-gun, Osaka (JP); Miyamoto, Tsumoru, c/o Minase Research Institute, Shimamoto-cho, Mishima-gun, Osaka (JP)
(74) Representative: Bentham, Stephen

(56) References cited:
- No relevant documents disclosed

## Description

### NOVEL POLYPEPTIDES AND DNAs ENCODING THEM

The present invention is related to new polypeptides which a glioblastoma cell line generates, and DNAs encoding the said polypeptides.

The cells which make up a brain may be largely grouped into neuron and glial cells. Neurons play a major role in the transmission and treatment of information in a brain.

On the other hand, glial cells support the action of neurons. Specifically glial cells are associated with protection and support of neurons, formation of myelin sheath, formation of blood brain barrier, delivery of nutrition to neurons and metabolism of neurotransmitters. They are further associated with the control of proliferation and differentiation of neurons when the brain is growing, etc. There are various kinds of glial cells including astrocytes, oligo-dendrocytes and microglias in the central nervous system, and Schwann cells and mantle cells in the peripheral nervous system. Ependymal cells existing in the cerebral ventricular endothelium are also glial cells.

Recently, many humoral factors for the differentiation, proliferation and growth of neurons and glial, have been found, and most of them are generated by glial or tumorous glial cells (referred as glia etc. as a whole hereafter).

For example, nerve growth factor (NGF, m.w. ca. 13.5 kd (human)), brain-derived neurotrophic factor (BDNF, m.w. ca. 13.5 kd (human)), neuroblastoma growth inhibitory factor (NGIF, m.w. ca. 5 kd (human)) and glia-derived neurotrophic factor (m.w. ca. 500 kd and ca. 110 kd (both rats)), which all are known as factors acting on neuron, are generated from astrocytes. Further, glia-derived glial growth inhibitory factor (GdGGIF, m.w. more than 100 kd (rats)) and platelet-derived growth factor (PDGF, m.w. ca. 32 kd (human)), both acting on glia, are also generated from astrocytes. Furthermore, glia-derived neurotrophic factor (GdNTF, m.w. ca. 43 kd (rats)) exhibiting a neurotrophic activity and a proliferating activity on glia, glia maturation factor (GMF, m.w. ca. 16.5 kd (bovine)) exhibiting a neurotrophic activity and a stimulatory activity on proliferation and differentiation of glioblastoma, cilially neurotrophic factor (CNTF, m.w. ca. 23 kd (rabbits and rats)) exhibiting an activity of life maintenance of ganglion cells, an inhibitory activity of proliferation of sympathetic ganglion cells, a stimulatory activity of differentiation of sympathetic ganglion cells and astrocytes, and fibroblast growth factor (FGF, m.w. ca. 16 to 17 kd (human)) exhibiting stimulatory activity of proliferation of neurons and astrocytes, all are generated from astrocytes. Schwann cell-derived neuron growth factor (SchNTF, m.w. more than 8 kd (rats)) acting on neuron is generated from Schwann cells (see PROTEIN, NUCLEIC ACID AND ENZYME, vol.36 (No. 7), p.260, 1991 in detail). It has also been found that interleukines (IL-1, IL-6 etc.) which are believed to be mainly secreted by immunocytes are also secreted from glial.

The amino acid sequences of most of these factors have been revealed. They are of interest not only for pure research but also for the research and development of pharmaceuticals.

The present inventors have sought to find novel factors (polypeptides) which glia etc. generate.

Conventional methods to obtain a particular polypeptide or a DNA encoding it, generally utilize methods in which an intended biological activity is confirmed in a tissue or in a cell medium, the active polypeptide is isolated and purified and then a gene encoding the polypeptide is cloned, or utilise methods of expression-cloning with the guidance of the biological activity.

However, physiologically active polypeptides in the living body have often many different kinds of activity. Therefore, it is increasingly found that after a gene is cloned, the gene is identical to one encoding a polypeptide already known. Also, glia generates only a very small amount of most factors and this makes it difficult to isolate and to purify a factor and to confirm its biological activity.

More recently, sequencing technology has been,rapidly developed, and methods based on "Reverse Genetics" for characterizing the function of a gene from the sequence of the gene, have also been greatly developed.

The present inventors attempted to find novel polypeptides by using these methods. That is, a series of experiments were carried out by isolating mRNA from glia etc, obtaining cDNA by using mRNA thus obtained, elucidating the nucleotide sequence of a cDNA and deducing the corresponding amino acid sequence. DNA thus obtained was transformed into a mammalian host cell to express a novel polypeptide as recombinant protein which was then purified. Further, amino acid-sequencing of the protein was carried out and the sequence has been confirmed to be identical to that deduced from the cDNA. Further, the amino acid sequence of the N-terminal end of its mature protein has been identified. In this manner, the present inventors have succeeded in identifiying a previously unidentified secretable polypeptide and DNA encoding it, to complete the present invention.

There was no polypeptide having an amino acid sequence which is identical to, or has high homology to, that of the polypeptide of the present invention, when the amino acid sequence of the polypeptide was compared by a computer to known sequences in the Swiss Prot database (Swiss Prot Release 20). Furthermore, there was no nucleotide sequence which is identical to, or has high homology to, that encoding the polypeptide of the present invention, when the DNA sequence was compared by a computer to known sequences in GenBank database (GenBank Release 70.0). Therefore, the polypeptide of the present invention is considered to be quite novel.

### Brief description of the drawings

Figure 1 shows the construction of plasmid vector, pVfCS-1.

Figure 2 shows the construction of a recombinant DNA into which cDNA derived from human glioblastoma cell line T98G is inserted.

Figure 3 shows a hydrophobicity profile of the polypeptide of the present invention.

Figure 4 shows the construction of an expression vector, pUC-SRαML1.

Figure 5 is a photograph showing patterns of SDS-PAGE in Example 10.

The present invention thus provides a polypeptide having the amino acid sequence shown in Seq. ID. No. 1 or 3 in substantially purified form, homologues thereof having at least 90% homology with the amino acid sequence shown in SEQ ID No. 1 or 3 over a region of at least 30 contiguous amino acids, or a fragment of the sequence, or a fragment of such a homologue comprising at least 20 amino acids. The invention further provides a DNA coding for such a polypeptide. A further embodiment of the invention provides a DNA having a nucleotide sequence shown in SEQ ID No. 1, 3 or 5 and fragments thereof comprising at least 20 nucleotides capable of selectively hybridizing to SEQ ID No. 1, 3 or 5.

The present invention in particular relates to:
(1) a polypeptide having an amino acid sequence shown in SEQ ID NO. 1,
(2) a DNA encoding the polypeptide described above (1),
(3) a DNA having a nucleotide sequence shown in SEQ ID NO. 1,
(4) a polypeptide having an amino acid sequence shown in SEQ ID NO. 3,
(5) a DNA encoding the polypeptide described above (4),
(6) a DNA having a nucleotide sequence shown in SEQ ID NO.3, and
(7) a DNA having a nucleotide sequence shown in SEQ ID NO.5.

A polypeptide of Seq. ID No. 1 or 3 in substantially purified form will generally comprise the polypeptide in a preparation in which more than 90%, eg. 95%, 98% or 99% of the polypeptide in the preparation is that of the Seq. ID No. 1 or 3.

A polypeptide homologue of the Seq. ID No. 1 or 3 will be generally at least 90% and preferably at least 95% homologous to the polypeptide of Seq. ID No. 1 or 3 over a region of at least 30, preferably at least 40, for instance 60 or 100 contiguous amino acids. Such polypeptide homologues will be referred to below as a polypeptide according to the invention.

Fragments of Seq. ID No. 1 or 3 or its homologues will be at least 20, for example 25, 30, 40, 50 or 60 amino acids in length, and are also encompassed by the term "a polypeptide according to the invention" as used herein. Particular fragments of the polypeptides of the invention are fragments of Seq. ID. No. 3 which include Seq. ID. No. 1, or a homologue thereof.

A DNA capable of selectively hybridizing to the DNA of Seq. ID No. 1, 3 or 5 will be generally at least 70%, preferably at least 80 or 90% and more preferably at least 95% homologous to the DNA of Seq. ID No. 1, 3 or 5 over a region of at least 20, preferably at least 30, for instance 40, 60 or 100 or more contiguous nucleotides. Such DNA will be encompassed by the term "DNA according to the invention".

DNA according to the invention may be used to produce a primer, eg. a PCR primer, a probe eg. labelled by conventional means using radioactive or non-radioactive labels, or the DNA may be cloned into a vector. Such primers, probes and other fragments of the DNA of Seq. ID No. 1, 3 or 5 will be at least 20, for example 25, 30 or 40 nucleotides in length, and are also encompassed by the term "DNA according to the invention" as used herein.

DNA according to the invention may be produced recombinantly, synthetically, or by any means available to those of skill in the art.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promotor. The vector may contain one or more selectable marker genes, for example an ampicillin resistance gene. The vector may be used in vitro, for example for the production of RNA corresponding to the DNA, or used to transform a host cell.

A further embodiment of the invention provides host cells transformed or transfected with the vectors for the replication and expression of DNA according to the invention, including the DNA Seq. ID No. 1, 3 or 5 or the open reading frame thereof. The cells will be chosen to be compatible with the vector and may for example be bacterial, yeast, insect or mammalian.

DNA according to the invention may also be inserted into the vectors described above in an antisense orientation in order to provide for the production of antisense RNA (DNA). Antisense RNA (DNA) may also be produced by synthetic means. Such antisense RNA (DNA) may be used in a method of controlling the levels of a polypeptide of the invention in a cell.

A further embodiment of the invention provides a method of producing a polypeptide which comprises culturing host cells of the present invention under conditions effective to express a polypeptide of the invention. Preferably, in addition, such a method is carried out under conditions in which the polypeptide of the invention is expressed and then secreted from the host cells.

The invention also provides monoclonal or polyclonal antibodies to a polypeptide according to the invention. The invention further provides a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, or an antibody thereof, in association with a pharmaceutically acceptable diluent or carrier.

The invention also provides a polypeptide according to the invention or an antibody for use in a method of therapy or diagnosis on a human or animal body.

It is known that most cytokines and growth factors, as well as a number of membrane proteins, have a signal peptide on their N-termini. The signal peptide is a highly hydrophobic region immediately downstream of the translation initiation amino acid Met. It was confirmed that the polypeptide of the present invention has a signal peptide which is located from methionine (Met) at the 1st position to serine (Ser) at the 23rd position in the amino acid sequence shown in SEQ ID NO. 3 (see Example 11). The essential sequence for biological activity (the mature protein sequence) is the amino acid sequence where the signal peptide is removed from the amino acid sequence shown in SEQ ID No. 3 i.e. the sequence corresponding to SEQ ID No. 1. The signal peptide is not required for biological activity.

The polypeptides of the present invention include fragments in which,part of the amino acid sequence is lacking (e.g., a polypeptide comprised of only essential sequence for revealing a biological acitivity in a mature protein), homologues in which a part of the amino acid sequence is replaced by other amino acids (e.g., those replaced by an amino acid having a similar property) and homologues in which other amino acids are added or inserted into a part of their amino acid sequence, as well as those having the exact amino acid sequence shown in SEQ ID Nos. 1 and 3.

As is known well, there are from one to six different codons which can encode each amino acid (for example, one kind of codon for Met, and six kinds of codon for leucine (Leu) are known). Accordingly, the nucleotide sequence of DNA can be changed in order to encode the polypeptide having the same amino acid sequence.

The DNA's of the present invention, specified in (2) and (5) include every nucleotide sequence encoding polypeptides shown in SEQ ID NOs. 1 and 3, as well as fragments and homologues thereof. There is a probability of improving a yield of production of a polypeptide by changing a nucleotide sequence.

The DNAs specified in (3) SEQ ID No.1 and (6) SEQ ID No. 3 are the specified embodiments which have been isolated as the natural DNA's encoding the polypeptide of SEQ ID Nos. 1 and 3.

The DNA (7) shown in SEQ ID No. 5 is the sequence of the DNA specified in (6) with a non-translational region.

The DNA having a nucleotide sequence shown in SEQ ID NO. 5 may be prepared according to the following methods, that is,
(i) by isolating mRNA from a cell line which secretes the polypeptide of the present invention (e.g., human glioblastoma cell line),
(ii) by preparing first strand (single stranded DNA) from mRNA thus obtained, followed by preparing second strand (double stranded DNA) (synthesis of cDNA),
(iii) by inserting cDNA thus obtained into a proper plasmid vector,
(iv) by transforming host cells with the recombinant DNA thus obtained (preparation of cDNA library),
(v) by random-cloning on a large scale from cDNA library thus obtained, followed by sequencing about 300 bases from 5' end of each clone, and
(vi) by sequencing complete length of a clone which has a novel base sequence.

Explained in detail, step (i) may be carried out in accordance with the method of Okayama, H. *et al.* (described in *Methods in Enzymology,* vol. 154, p 3, 1987) after a human glioblastoma cell line is stimulated by a proper stimulant (e.g., IL-1 etc.). Examples of the cells which secrete the polypeptide of the present invention is preferably human glioblastoma cell line T98G (ATCC strain No., CRL-1690). Steps (ii), (iii) and (iv) are a series of steps for preparing cDNA library, and may be carried out in accordance with the method of Gubler & Hoffman (*Gene*, vol. 25, p. 263, 1983) with a slight modification. As examples of the plasmid vector used in the step (iii), many vectors functioning in an *E. coli* strain (e.g., pBR 322) and in a *Bacillus subtilis* (e.g., pUB 110) are known, and pVfCS-1 (described in detail in the following Example) prepared from pGEM-37f(+) (3,199 bp, manufactured by Promega Corp.) which functions in an *E. coli,* may be preferably used. As examples of host used in the step (iv), many cells are already known. Any cells may be used, and DH5 competent cell which has been prepared in accordance with the method described in *Gene*, vol. 96, p. 23, 1990, may be preferably used. The cloning in the step (v) may be carried out by methods known *per se* and the sequencing may be carried out in accordance with the method of Maxam-Gilbert or the dideoxy termination method. The step (vi) may be carried out in accordance with the method described in *Molecular Cloning* (written by Sambrook, J., Fritsch, E. F. and Maniatis, T., published by Cold Spring Harbor Laboratory Press in 1989).

As the following step, it is necessary to examine whether or not the DNA thus obtained codes right a secretory protein. The examination requires:
(I) the conversion of the DNA sequence into the amino acid sequence in a possible frame,
(II) the preparation of hydrophobicity profile from the amino acid sequence thus obtained, followed by confirmation of the existence of a highly hydrophobic region just after the translation initiation codon (ATG) (secretory proteins have highly hydrophobic signal peptides on their N-termini), and then
(III) the confirmation that the DNA thus obtained covers complete or almost complete length of intact mRNA. These confirmation may be carried out after the step (vi) hereinbefore described, and effectively between the step (v) and the step (vi).

In the above examination, the step (II) may be carried out by analyzing using a computer making use of a hydro table of Kyte, J. and Doolittle, R. F., and an available software, e.g., DNASIS (Hitachi Software Engineering Co., Ltd.). The Step (III) may be carried out by northern analysis.

Once the nucleotide sequences shown in SEQ ID NOs. 2, 4 and 5 are determined, DNA of the present invention may be obtained by chemical synthesis, by PCR method or by hybridization making use of a fragment of DNA of the present invention, as a probe. Furthermore, DNA of the present invention may be obtained in a desired amount by transforming with a vector DNA inserted a DNA of the present invention into a proper host, followed by culturing the transformant.

The polypeptides of the present invention (shown in SEQ ID NOs. 1 and 3) may be prepared by:
(1) isolating and purifying from an organism or a cultured cell,
(2) chemically synthesizing, or
(3) using a skill of biotechnology, preferabaly, by the method described in (3).

Examples of expression system when preparing a polypeptide by using a skill of biotechnology, is, for example, the expression system of bacteria, yeast, insect cell and mammalian cell.

For example, the expression in *E. coli* may be carried out by adding the initiation codon (ATG) to 5' end of a DNA encoding a mature protein (e.g., DNA encoding a nucleotide sequence shown in SEQ ID NO. 2), connecting the DNA thus obtained to the downstream of a proper promoter (e.g., trp promoter, lac promoter, λP_{L} promoter, T7 promoter etc.), and then inserting it into a vector (e.g., pBR322, pUC18, pUC19 etc.) which functions in an *E. coli* strain to prepare an expression vector. Then, an *E. coli* strain (e.g., *E. coli* DH1 strain, *E. coli* JM109 strain, *E. coli* HB101 strain, etc.) which is transformed with the expression vector thus obtained may be cultured in a proper medium to obtain the desired polypeptide. When a signal peptide of bacteria (e.g., signal peptide of pel B) is utilized, the desired polypeptide may be also secreted in periplasm. Furthermore, a fusion protein with other polypeptide may be also produced easily.

Furthermore, the expression in a mammalian cell may be carried out, for example, by inserting the DNA shown in SEQ ID NO. 5 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transforming a proper mammalian cell (e.g., monkey COS-7 cell, chinese hamster CHO cell, mouse L cell etc.) with the expression vector thus obtained, and then culturing the transformant in a proper medium to get a desired polypeptide in the culture medium. The polypeptide thus obtained may be isolated and purified by conventional biochemical methods.

The polypeptide of the present invention is synthesized in and secreted from a human glioblastoma cell line, and therefore, may possess biological activities relating to differentiation, proliferation and growth, of glias or neurons, relating to the function of immunity and relating to proliferation and growth, of tumors. The polypeptide of the present invention may be useful for the prevention of or in the treatment of aplasia or abnormal proliferation, of glias or neunons, depression or enhancement of immunological activity, or tumors etc.

Further, polyclonal or monoclonal antibody against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various use or for diagnosis of and in the treatment of gene diseases (the treatment of gene defect disease and the treatment by inhibiting expression of the polypeptide by antisense DNA (RNA), and the like). Further, genomic DNA may be isolated by using the DNA of the present invention as a probe. Similarly, it is possible to isolate genes having high homology to the DNA of the present invention in human or those of other species.

### Examples

The following examples are illustrated, but not limit, the present invention.

### Example 1: Construction of vector for use in the preparation of cDNA library

A plasmid vector, pGEM-3Zf(+) (3,199 bp, manufactured by Promega Corp.) was digested with *Hin*dIII, followed by Klenow treatment and circularized again. The necessary amount of this plasmid was recovered from culture of *E. coli* transformed with the plasmid. Next, an *AatI*I-*Nde*l fragment was cut out from the plasmid, and the resulting linear plasmid fragment was smoothended using T4 polymerase. After ligating the thus treated termini with a *Hind*III linker, the resulting fragment was digested with *Hind*III, circularized again and then transformed into an *E. coli* strain to recover plasmid.

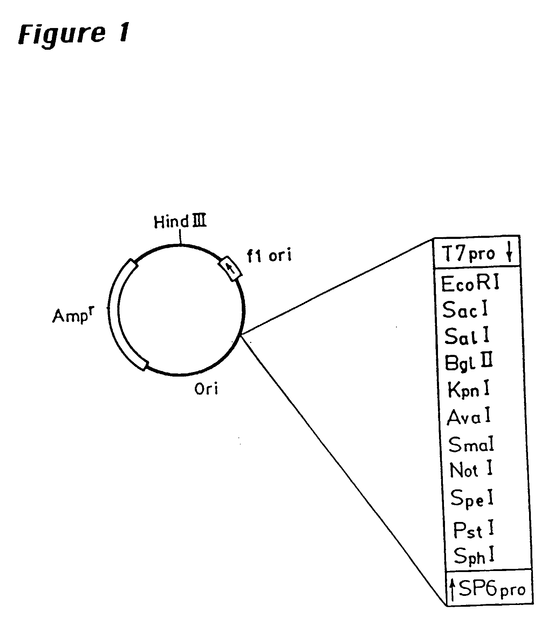

Thereafter, a *Sac*l*-Pst*l portion in the polylinker of the plasmid was replaced by a synthetic polylinker shown below. The plasmid vector thus constructed (see Fig. 1) was named pVfCS-1.

The pVfCS-1 has the following characteristic properties as a multi-purpose plasmid vector.
1. Okayama-Berg method and Gubler-Hoffman method can be applied.
2. Plasmid yield per cultured cells is high.
3. Single-stranded DNA can be prepared.
4. A cDNA insert can be cut out easily.
5. Preparation of a deletion mutant for sequencing use can be made easily.
6. *In vitro* transcription can be made.

### Example 2: Isolation and purification of mRNA

A total of 3 x 10⁷ cells of a human glioblastoma cell line T98G (ATCC strain No., CRL-1690) were stimulated for 4 hours with 100 units/ml of human IL-1β, subsequently isolating mRNA in accordance with the method of Okayama, *H. et al. (Methods in Enzymology*, vol. 154, p. 3, 1987) as described below.

That is, the stimulated cells were solubilized with a 5.5 M GTC solution (5.5 M guanidine thiocyanate, 25 mM sodium citrate, 0.5% sodium lauryl sarcosine), and the resulting cell lysate was laid on a cushion of a cesium trifluoroacetate (CsTFA) solution having a density of 1.51 and centrifuged (120,000 x g, 20 hours) to recover 1.26 mg of total RNA in the resulting pellet. Thereafter, the RNA sample was passed twice through an oligo(dT)-cellulose column to purify and recover 46 µg of poly(A)^{**+**}RNA.

### Example 3: Preparation of cDNA library

A cDNA library was prepared in accordance with the method of Gubler & Hoffman (*Gene,* vol. 25, p. 263, 1983) with a slight modification.

A first strand was synthesized from the poly(A)⁺ RNA (5 µg) prepared in Example 2, using a reverse transcriptase and an oligo(dT) primer having a *Not*I site. After synthesizing a second strand and carrying out *Sal*I adaptor ligation and *Not*I digestion, the adaptor and the primer were removed by gel filtration column chromatography using a column packed with Sephacryl S-500HR (available from Pharmacia), thereby recovering a fraction containing 820 ng of cDNA.

The above cDNA synthesizing step was effected making use of a kit (Super Script System, available from BRL).

Separately from this, a vector was prepared by subjecting the pVfCS-1 obtained in Example 1 to complete digestion with *Not*I, digesting the product further with *Sal*I, subjecting the resulting digest to 0.8% agarose gel electrophoresis to cut out a band of interest and then purifying the vector of interest making use of a kit for glass powder method use (GENECLEAN II, available from BIO 101).

After subjecting the thus prepared cDNA and vector to ligation, the resulting product was transfected into DH5 competent cells which have been prepared in accordance with the method of Inoue, H. *et al*. (*Gene*, vol. 96, p. 23, 1990). As the results, a cDNA library containing 6 x 10⁵ independent clones with an average length of 1.5 kb was obtained.

### Example 4: Cloning and Sequencing

Using the cDNA library prepared in Example 3, clones were plated on LB-broth agar containing ampicilin with a density of 300 colonies/dish having a diameter of 10 cm. Cloning was carried out by picking up the colonies at random. Each of the colonies was cultured overnight in 3 ml of LB-broth. A 200 µl portion of the resulting culture was mixed with dimethylsulfoxide (DMSO, final concentration of 7%) and stored at -80°C, and the remaining portion of the culture was used for the isolation of plasmid. The purification of plasmid was carried out by the usual way.

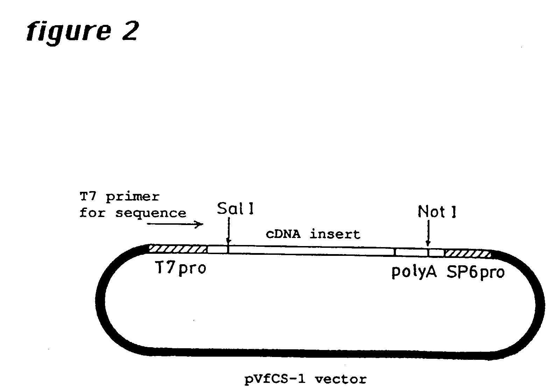

Since the plasmid has a structure shown in Fig. 2, its nucleotide sequence can be read from the 5' end of the cloned cDNA when sequencing is carried out using T7 primer.

DNA sequencing was carried out in accordance with a cycle sequence method based on the dideoxy termination method of Sanger, F. *et al*., using a fluorescence dye terminator of ABI (Applied Biosystems Inc.). Reading of the sequence was carried out using a DNA sequenser of ABI (Model 373A).

In this way, a nucleotide sequence having a length of about 300 bases from 5' end of each cDNA was obtained.

### Example 5: Analysis of partial sequence data

Using the FASTA program of Lipman, D. J. and Pearson, W. R., the nucleotide sequence obtained in Example 4 was searched for its homology with every nucleotide sequence contained in known data bases (GenBank and EMBL). As the results, a clone having an unknown sequence was identified. The unknown nucleotide sequence was converted into amino acid sequences in possible three frames.

Next, hydrophobic and hydrophilic profiles of the amino acid sequences deduced from their corresponding three frames were analyzed graphically using a computer making use of a hydro table of Kyte, J. and Doolittle, R. F. In this instance, DNASIS (Hitachi Software Engineering Co., Ltd.) was used as a computer software.

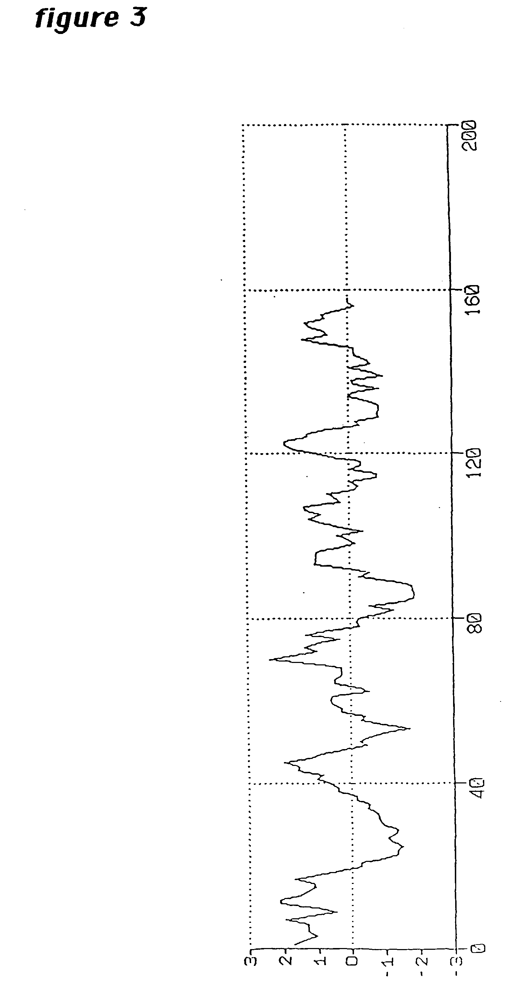

It is known that most of cytokines and growth factors, as well as a number of membrane proteins, have signal peptides on their N-termini. Signal peptide is a highly hydrophobic region immediately downstream of the translation initiation amino acid Met. In a nucleotide sequence, it is located just after the initiation codon (ATG) of its coding region. In consequence, a cDNA having a putative signal peptide-containing protein was screened on the basis of data on the open reading frame of the clone having unknown sequence and on the hydrophobic and hydrophilic properties of the amino acid sequence. As the results, a clone of interest (clone No. TG0077) was found which showed signal peptide-specific nature in one of its three possible amino acid sequence frames. (See Fig. 3 which shows a hydrophobicity profile of the total amino acid sequence. In the figure, the "+" side is a highly hydrophobic region and the "-" side is a region having weak hydrophobicity.)

It is possible however that the cloned cDNA does not cover complete length of mRNA. If it does not cover the complete length, it is less possible that the clone contains the N-terminal amino acid sequence moiety.

In consequence, northern analysis was carried out in order to determine if the clone TG0077 has complete length or not. That is, poly(A)⁺RNA which has been extracted and purified from a glioblastoma cell line was subjected to electrophoresis and then blotting on nylon membrane. When hybridization was carried out using a TG0077 cDNA insert as a probe, a single band was found at a position corresponding to about 700 bp. Since the TG0077 cDNA insert had a size of about 700 bp, it was confirmed that the TG0077 clone was a almost complete length cDNA.

### Example 6: Determination of complete sequence of the cDNA and open reading frame

The complete length cDNA sequence was determined by means of random sequencing in accordance with the method disclosed in *Molecular Cloning* (Sambrook, J., Fritsch, E. F. and Maniatis, T., Cold Spring Harbor Laboratory Press, 1989).

Plasmid was recovered from the TG0077 clone to isolate and purify a cDNA insert. The insert thus purified was subjected to ligation and fragmentation, followed by smooth-ending of both termini of the resulting DNA fragment with T4 polymerase, thereby recovering a DNA fragment of about 400 bp in length by agarose gel electrophoresis. The thus obtained DNA fragment was cloned into a *Sma*I site of a plasmid vector BLUESCRIPT II (available from Stratagene) and then transfected into an *E. coli* strain. A total of 20 colonies were picked up at random to prepare 20 corresponding plasmid DNA samples (every of them contained a TG0077 cDNA fragment as an insert) which were subsequently subjected to DNA sequencing. DNA sequencing and sequence reading were carried out in the same manner as the procedure described in Example 4. Sequence data of the TG0077 cDNA fragment were arranged into a continued sequence making use of a DNA sequence connection program of DNASIS, thereby obtaining a base sequence shown in SEQ ID NO. 5. An open reading frame was determined based on the complete length cDNA sequence data to deduce corresponding amino acid sequence, with the results shown in SEQ ID NO. 3.

### Example 7: Construction of plasmid vector for use in the preparation of expression vector

A vector, called pcD-SRα296, constructed by Takebe *et al. (Mol. Cell. Biol.,* vol. 8, p. 966, 1988) is an excellent expression vector for use in animal cells, which contains a promoter system (SRα) consisting of SV40 early promoter, R region of LTR of HTLV-I and a part of U5 sequence. This vector, however, has disadvantages in that (1) it has only one insert-cloning site, namely an *Eco*RI site, and (2) its recovery yield from *E. coli* is small because of the use of pBR322 vector as its basic structure. In consequence, an attempt was made to modify the pcD-SRα296 vector in the following manner, with the aim of constructing a modified vector containing multiple insert-cloning sites and having a basic structure derived from pUC19 which is effective in recovering the resulting modified vector in a high yield from *E. coli.*

The pcD-SRα296 vector (provided by Dr. Takebe at National Institute of Health) was digested with *Sal*I and the resulting digest was subjected to agarose gel electrophoresis to separate and recover a 1.7 kbp fragment containing the SRα promoter, subsequently smooth-ending the thus obtained fragment by Klenow treatment.

Separately from this, pUC19 vector was digested with *Nde*I and *Hin*dIII and the resulting digest was subjected to agarose gel electrophoresis to separate and recover a 2.4 kbp fragment containing Amp^{r} and pUCori regions, subsequently smooth-ending the thus obtained fragment by Klenow treatment and thereafter removing the 5'-end phosphoric acid group by BAP (bacterial alkaline phosphatase) treatment.

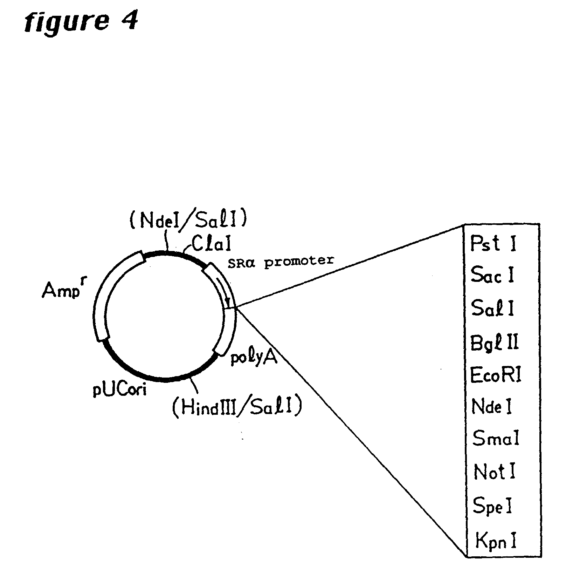

The thus prepared 1.7 kbp fragment containing the SRα promoter was ligated with the pUCori-containing 2.4 kbp fragment to make them into a circular form, thereby effecting construction of a new vector. A *Pst*I-*Kpn*I fragment was removed from the thus obtained vector and replaced with the following synthetic polylinker. The plasmid vector constructed in this manner (about 3.9 kbp, see Fig. 4) was named pUC-SRαML1.

### Example 8: Construction of expression vector

A cDNA insert was cut out using *Sal*I and *Spe*I from plasmid which has been isolated from the TG0077 clone prepared in Example 5, and the cDNA insert was inserted into a *Sal*I-*Spe*I site of the pUC-SRαML1 obtained in Example 7, thereby obtaining an expression vector, pUC-SRαML1-TG0077A.

Separately from this, oligodeoxynucleotides represented by the following formulae: and were synthesized, and the TG0077 plasmid was subjected to PCR (polymerase chain reaction) using the thus synthesized oligodeoxynucleotides as primers. The thus obtained PCR fragment contains cDNA which encodes a protein molecule consisting of the TG0077 protein and six additional histidine (His) residues attached to its C-terminal end. The PCR fragment was digested with *Sal*I and *Spe*I, and the resulting digest was separated and purified and then inserted into the *Sal*I-*Spe*I site of the pUC-SRαML1 prepared in Example 7 to obtain an expression vector pUC-SRαML1-TG0077B.

Each of the thus constructed pUC-SRαML1-TG0077A and pUC-SRαML1-TG0077B plasmids was transfected into an *E. coli* strain DH5, recovered from a 100 ml culture of the resulting transformant and then purified by CsCl density gradient centrifugation twice. In this manner, 592 µg and 546 µg of the respective plasmids were recovered.

### Example 9: Expression in COS cells

Each of the plasmid DNA preparations pUC-SRαML1, pUC-SRαML1-TG0077A and pUC-SRαML1-TG0077B was introduced into COS-7 cells (*Cell*, vol. 23, p. 175, 1981) by means of the diethylaminoethyl (DEAE) dextran method (*J. Immunology*, vol. 136, p. 4291, 1986).

That is, about 1.8 x 10⁶ COS-7 cells were inoculated into a 225 cm² capacity flask (manufactured by Corning) together with 50 ml of a liquid culture medium (Dulbecco's modified MEM medium supplemented with 10% decomplemented fetal bovine serum). After overnight incubation in a carbon dioxide incubator (37°C, 5% CO₂) and subsequent removal of the culture supernatant, 12 ml of a DNA cocktail (Dulbecco's modified MEM medium supplemented with 15 µg of each plasmid DNA, 50 mM Tris-HCl buffer (pH 7.4) and 400 µg/ml of DEAE-dextran) was added to each flask and culture was carried out for 3 hours at 37°C in an atmosphere of 5% CO₂. Thereafter, the DNA cocktail was replaced by 15 ml of a chloroquine solution (Dulbecco's modified MEM medium supplemented with 150 µM chloroquine and 7% decomplemented fetal bovine serum), followed by additional 3 hours of culture.

After removing the chloroquine solution, the aforementioned liquid culture medium (50 ml) was added to each of the resulting flasks which were then incubated at 37°C in an atmosphere of 5% CO₂ for 72 hours to find growth of the cells in each flask into almost monolayer form. After removing the culture supernatant, the cells in each flask were washed with a serum-free liquid culture medium (trade name, SFM-101; available from Nissui Pharmaceutical Co., Ltd.) and then supplied with 75 ml of the same serum-free liquid culture medium, and the culturing was continued for another 72 hours. Thereafter, the resulting culture supernatant was recovered and filtered through a membrane filter (trade name, STERIVEX-GS; available from Millipore Corp.) to remove the cells and cell debris. The thus obtained culture supernatant samples were stored at 4°C for future use. A culture supernatant of COS cells which have been transformed with plasmid containing the TG0077 cDNA insert is expected to contain expressed and secreted mature protein moiety of a polypeptide which corresponds to TG0077.

### Example 10: Confirmation of expression

A 2 ml portion of each of the culture supernatants of transformed COS cells obtained in Example 9 was concentrated to a volume of 100 µl using a centrifugal concentration filter (trade name, Centricon-10; available from Millipore Corp.). A 1 µl portion of each of the thus concentrated samples was mixed with the same volume of a loading buffer (0.125 M Tris-HCl buffer (pH 6.8), 4% sodium dodecyl sulfate and 30% glycerol) for SDS-PAGE (sodium dodecyl sulfate polyacrylamide gel electrophoresis) use, and the mixture was treated at 90°C for 3 minutes and then subjected to SDS-PAGE.

In the case of the TG0077B protein having His hexamer introduced to the C-terminus of the protein, not only its corresponding COS cell culture supernatant but also its purified product were subjected to the SDS-PAGE analysis.

Purification of the protein was carried out by means of a metal chelate affinity chromatography (*Biotechnology*, vol. 9, p. 273, 1991), making use of the function of His to form complex compounds with various transition metal ions. That is, a culture supernatant (350 ml) obtained from COS cells was mixed with a sodium chloride aqueous solution in such an amount that the final concentration of the salt became 1 M, and the resulting mixture was applied to a column packed with 4 ml of a zinc-linked chelating Sepharose (trade name, Chelating Sepharose Fast-Flow; available from Pharmacia) to adsorb the protein to the resin. The column was washed with 50 mM phosphate buffer (pH 7.0) containing 1 M sodium chloride aqueous solution (40 ml), and the protein retained in the column was eluted with 50 mM phosphate buffer (pH 7.0) containing 1 M sodium chloride aqueous solution and 0.4 M imidazole. Thereafter, 12 ml of the resulting eluate was concentrated to a volume of 100 µl, and a 1 µl portion of the concentrated sample was subjected to SDS-PAGE analysis.

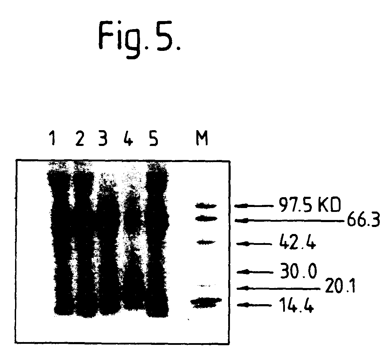

The SDS-PAGE analysis was carried out making use of a Phast System (available from Pharmacia) using a gel (Phast Gel™ SDS 10/20 gradient, available from Pharmacia). Results of silver staining are shown in Fig. 5.

In the figure, lane 1 indicates a concentrated culture supernatant of COS cells transformed with pUC-SRαML1, lane 2 indicates a concentrated culture supernatant of COS cells transformed with pUC-SRαML1-TG0077B, lane 3 indicates a non-adsorbed fraction obtained by the column purification of the culture supernatant of COS cells transformed with pUC-SRαML1-TG0077B, lane 4 indicates a fraction eluted with imidazole at the time of the same column purification, lane 5 indicates a concentrated culture supernatant of COS cells transformed with pUC-SRαML1-TG0077A and lane M represents molecular weight markers.

A band which corresponds to a molecular weight of about 15 to 16 kD was found on the lanes 2, 4 and 5 but not on the lanes 1 and 3. This band was found to be originated from the expressed TG0077 protein, because its position coincided with a molecular weight calculated from the amino acid sequence of the TG0077 protein. Recovered amount of the protein purified by the metal chelate affinity column chromatography was estimated to be about 20 µg based on the comparison of the silver-stained SDS-PAGE band with a standard band of human IL-4.

### Example 11: Amino acid sequencing of expressed protein

Amino acid sequence of the purified protein obtained by the metal chelate affinity column chromatography was determined starting from its N-terminal end, using a gas phase sequenser in accordance with the method of Matsudaira *et al. (J. Biol. Chem*., vol. 262, p. 10035, 1987).

Firstly, approximately 100 pmol of TG0077B protein (purified product obtained in Example 10) was subjected to 1 hour of SDS-PAGE at 60 mA using 10 to 20% polyacrylamide gradient gel (available from Daiichi Pure Chemical Co., Ltd.). After completion of the electrophoresis, the resulting gel was equilibrated with a blotting buffer [10 mM CAPS (3-cyclohexylamino-1-propane sulfonate) and 10% methanol] and then subjected to 2 hours of electro-blotting at 0.4 A, using a blotting apparatus (available from Marisol), on a PVDF (polyvinylidene difluoride) membrane (trade name, Pro Blott; available from ABI) which has been equilibrated with the same blotting buffer. After completion of the blotting, the resulting PVDF membrane was washed three times with distilled water, stained for 1 minute with 0.1% (w/v) Coomassie Blue and then decolorized by 50% methanol. Thereafter, a band of interest was cut out and applied to a protein sequenser (Model 470A, equipped with an on-line PTH analyzer Model 120A; available from ABI).

As the results, the sequence from the N-terminus was determined as follows.

Gly-X₁-Pro-Pro-X₂-Gln-X₃-Ser-X₄ ... ... ...

(In the above formula, X₁ to X₄ indicate that respective amino acid residues were not able to be judged clearly on the sequenser chart.). This sequence coincided with a downstream portion, starting at the 24 position amino acid residue, of the amino acid sequence deduced from the cDNA sequence. Based on these results, it was confirmed that the TG0077 protein has a signal peptide of 23 amino acid residues on its N-terminal side and the N-terminus of the mature protein is glycine (Gly).

Complete nucleotide sequence of the TG0077 cDNA and primary amino acid sequence of the TG0077 protein encoded by the nucleotide sequence are shown in SEQ ID NO. 6.

## Claims

1. A polypeptide having the amino acid sequence shown in SEQ ID No. 1 in substantially purified form, a homologue thereof having at least 90% homology with the amino acid sequence shown in SEQ ID No. 1 over a region of at least 30 contiguous amino acids, or a fragment of the sequence, or a fragment of such a homologue comprising at least 20 amino acids.

2. A polypeptide according to claim 1 having the amino acid sequence shown in SEQ ID No. 1.

3. A polypeptide comprising a polypeptide according to claim 1 or 2, having the amino acid sequence shown in SEQ ID No. 3 in substantially purified form, a homologue thereof having at least 90% homology with the amino acid sequence shown in SEQ ID No. 3 over a region of at least 30 contiguous amino acids, or a fragment of the sequence or a fragment of such a homologue comprising at least 20 amino acids.

4. A polypeptide according to claim 3 having the amino acid sequence shown in SEQ ID No. 3.

5. DNA encoding a polypeptide according to any one of the preceding claims.

6. DNA according to claim 5 having the nucleotide sequence shown in SEQ ID No. 1 or a fragment thereof comprising at least 20 nucleotides capable of selectively hybridizing to SEQ ID No. 1.

7. DNA according to claim 5 or 6 having the nucleotide sequence shown in SEQ ID No. 3 or a fragment thereof comprising at least 20 nucleotides capable of selectively hybridizing to SEQ ID No. 3.

8. DNA according to claim 5, 6 or 7 having the nucleotide sequence shown in SEQ ID No. 5 or a fragment thereof comprising at least 20 nucleotides capable of selectively hybridizing to SEQ ID No. 5.

9. A replication and expression vector comprising DNA according to any one of claims 4 to 8.

10. Host cells transformed or transfected with a replication and expression vector according to claim 9.

11. A method of producing a polypeptide which comprises culturing host cells according to claim 10 under conditions effective to express a polypeptide according to any one of claims 1 to 4.

12. A monoclonal or polyclonal antibody to a polypeptide according to any one of claims 1 to 4.

13. A pharmacuetical composition containing a polypeptide according to any one of claims 1 to 4 or an antibody according to claim 12 in association with a pharmacuetically acceptible deluant of carrier.

## Patentansprüche

1. Polypeptid mit der in der Sequenz mit der Identifizierungsnummer 1 dargestellten Aminosäuresequenz in im wesentlichen gereinigter Form, ein Homologes hiervon mit einer mindestens 90 % Homologie mit der in der Sequenz mit der Identifizierungsnummer 1 dargestellten Aminosäuresequenz über einen Bereich von mindestens 30 benachbarten Aminosäuren oder ein Fragment der Sequenz oder ein Fragment eines derartigen Homologen, das mindestens 20 Aminosäuren umfaßt.

2. Polypeptid nach Anspruch 1 mit der in der Sequenz mit der Identifizierungsnummer 1 dargestellten Aminosäuresequenz.

3. Polypeptid, umfassend ein Polypeptid nach Anspruch 1 oder 2, mit der in der Sequenz mit der Identifizierungsnummer 3 dargestellten Aminosäuresequenz in im wesentlichen gereinigter Form, ein Homologes hiervon mit einer mindestens 90 % Homologie mit der in der Sequenz mit der Identifizierungsnummer 3 dargestellten Aminosäuresequenz über einen Bereich von mindestens 30 benachbarten Aminosäuren oder ein Fragment der Sequenz oder ein Fragment eines derartigen Homologen, das mindestens 20 Aminosäuren umfaßt.

4. Polypeptid nach Anspruch 3 mit der in der Sequenz mit der Identifizierungsnummer 3 dargestellten Aminosäuresequenz.

5. DNA mit Kodierung für ein Polypeptid nach einem der vorhergehenden Ansprüche.

6. DNA nach Anspruch 5 mit der in der Sequenz mit der Identifizierungsnummer 1 dargestellten Nukleotidsequenz oder ein Fragment hiervon, das mindestens 20 Nukleotide mit der Fähigkeit zur selektiven Hybridisierung an die Sequenz mit der Identifizierungsnummer 1 umfaßt.

7. DNA nach Anspruch 5 oder 6 mit der in der Sequenz mit der Identifizierungsnummer 3 dargestellten Nukleotidsequenz oder ein Fragment hiervon, das mindestens 20 Nukleotide mit der Fähigkeit zur selektiven Hybridisierung an die Sequenz mit der Identifizierungsnummer 3 umfaßt.

8. DNA nach Anspruch 5, 6 oder 7 mit der in der Sequenz mit der Identifizierungsnummer 5 dargestellten Nukleotidsequenz oder ein Fragment hiervon, das mindestens 20 Nukleotide mit der Fähigkeit zur selektiven Hybridisierung an die Sequenz mit der Identifizierungsnummer 5 umfaßt.

9. Replikations- und Expressionsvektor, der DNA nach einem der Ansprüche 4 bis 8 umfaßt.

10. Mit einem Replikations- und Expressionsvektor nach Anspruch 9 transformierte oder transfizierte Wirtzellen.

11. Verfahren zur Herstellung eines Poypeptids durch Züchten von Wirtzellen nach Anspruch 10 unter Bedingungen, die zur Expression eines Polypeptids nach einem der Ansprüche 1 bis 4 führen.

12. Monoklonaler oder polyklonaler Antikörper zu dem Polypeptid nach einem der Ansprüche 1 bis 4.

13. Pharmazeutische Zubereitung, die ein Polypeptid nach einem der Ansprüche 1 bis 4 oder einen Antikörper nach Anspruch 12 in Verbindung mit einem pharmazeutisch akzeptablen Verdünnungsmittel oder Träger enthält.

## Revendications

1. Polypeptide ayant la séquence d'acides aminés présentée dans SEQ ID N°. 1 sous forme pratiquement purifiée, homologue de celui-ci ayant au moins 90 % d'homologie avec la séquence d'acides aminés présentée dans SEQ ID N°. 1 sur une région d'au moins 30 acides aminés contigus, ou fragment de la séquence, ou fragment de cet homologue, comprenant au moins 20 acides aminés.

2. Polypeptide conforme à la revendication 1, ayant la séquence d'acides aminés présentée dans SEQ ID N°. 1.

3. Polypeptide comprenant un polypeptide conforme à la revendication 1 ou la revendication 2, ayant la séquence d'acides aminés présentée dans SEQ ID N°. 3 sous forme pratiquement purifiée, homologue de celui-ci ayant au moins 90 % d'homologie avec la séquence d'acides aminés présentée dans SEQ ID N°. 3 sur une région d'au moins 30 acides aminés contigus, ou fragment de la séquence, ou fragment de cet homologue, comprenant au moins 20 acides aminés.

4. Polypeptide conforme à la revendication 3, ayant la séquence d'acides aminés présentée dans SEQ ID N°. 3.

5. ADN codant pour un polypeptide conforme à l'une quelconque des revendications précédentes.

6. ADN conforme à la revendication 5, ayant la séquence nucléotidique présentée dans SEQ ID N°. 1 ou fragment de celui-ci, comprenant au moins 20 nucléotides capables de s'hybrider sélectivement à SEQ ID N°. 1.

7. ADN conforme à la revendication 5 ou la revendication 6, ayant la séquence nucléotidique présentée dans SEQ ID N°. 3 ou fragment de celui-ci comprenant au moins 20 nucléotides, capable de s'hybrider sélectivement à SEQ ID N°. 3.

8. ADN conforme à la revendication 5, la revendication 6 ou la revendication 7, ayant la séquence nucléotidique présentée dans SEQ ID N°. 5 ou fragment de celui-ci comprenant au moins 20 nucléotides, capable de s'hybrider sélectivement à SEQ ID N°. 5.

9. Vecteur de réplication et d'expression comprenant de l'ADN conforme à l'une quelconque des revendications 4 à 8.

10. Cellule hôte transformée ou transfectée par un vecteur de réplication et d'expression conforme à la revendication 9.

11. Procédé de production d'un polypeptide qui comprend la culture de cellules hôtes conformes à la revendication 10 dans des conditions permettant l'expression d'un polypeptide conforme à l'une quelconque des revendications 1 à 4.

12. Anticorps monoclonal ou polyclonal contre un polypeptide conforme à l'une quelconque des revendications 1 à 4.

13. Composition pharmaceutique contenant un polypeptide conforme à l'une quelconque des revendications 1 à 4 ou anticorps conforme à la revendication 12 en association avec un diluant ou un véhicule acceptable du point de vue pharmaceutique.


### SEQUENCE LISTING

### (1) GENERAL INFORMATION:

(i) APPLICANT:
   (A) NAME: Ono Pharmaceutical Co Ltd
   (B) STREET: 1-5 Doshomachi 2-Chome
   (C) CITY: Osaka
   (E) COUNTRY: Japan
   (F) POSTAL CODE (ZIP): 541
(ii) TITLE OF INVENTION: Novel Polypeptides and DNAs Encoding Them
(iii) NUMBER OF SEQUENCES: 10
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(v) CURRENT APPLICATION DATA:
   APPLICATION NUMBER: EP 93301571.1

### (2) INFORMATION FOR SEQ ID NO: 1:

(i) SEQUENCE CHARACTERISTICS:
   (A) LENGTH: 399 base pairs
   (B) TYPE: nucleic acid
   (C) STRANDEDNESS: double
   (D) TOPOLOGY: linear
(ii) MOLECULE TYPE: cDNA
(ix) FEATURE:
   (A) NAME/KEY: CDS
   (B) LOCATION: 1..399
(xi) SEQUENCE DESCRIPTION: SEQ ID NO: 1:

### (2) INFORMATION FOR SEQ ID NO: 2:

(i) SEQUENCE CHARACTERISTICS:
   (A) LENGTH: 133 amino acids
   (B) TYPE: amino acid
   (D) TOPOLOGY: linear
(ii) MOLECULE TYPE: protein
(xi) SEQUENCE DESCRIPTION: SEQ ID NO: 2:
   Gly Ser Pro Pro Thr Gln Pro Ser Pro Ala Ser Asp Ser Gly Ser Gly
    1              5                  10                  15
   Tyr Val Pro Gly Ser Val Ser Ala Ala Phe Val Thr Cys Pro Asn Glu
               20                  25                  30
   Lys Val Ala Lys Glu Ile Ala Arg Ala Val Val Glu Lys Arg Leu Ala
           35                  40                  45
   Ala Cys Val Asn Leu Ile Pro Gln Ile Thr Ser Ile Tyr Glu Trp Lys
       50                  55                  60
   Gly Lys Ile Glu Glu Asp Ser Glu Val Leu Met Met Ile Lys Thr Gln
   65                  70                  75                  80
   Ser Ser Leu Val Pro Ala Leu Thr Asp Phe Val Arg Ser Val His Pro
                   85                  90                  95
   Tyr Glu Val Ala Glu Val Ile Ala Leu Pro Val Glu Gln Gly Asn Phe
               100                 105                 110
   Pro Tyr Leu Gln Trp Val Arg Gln Val Thr Glu Ser Val Ser Asp Ser
           115                 120                 125
   Ile Thr Val Leu Pro
       130

### (2) INFORMATION FOR SEQ ID NO: 3:

(i) SEQUENCE CHARACTERISTICS:
   (A) LENGTH: 468 base pairs
   (B) TYPE: nucleic acid
   (C) STRANDEDNESS: double
   (D) TOPOLOGY: linear
(ii) MOLECULE TYPE: cDNA
(ix) FEATURE:
   (A) NAME/KEY: CDS
   (B) LOCATION: 1..468
(xi) SEQUENCE DESCRIPTION: SEQ ID NO: 3:
   ATG CCG GCG CTG CTG CCT GTG GCC TCC CGC CTT TTG TTG CTA CCC CGA      48
   Met Pro Ala Leu Leu Pro Val Ala Ser Arg Leu Leu Leu Leu Pro Arg
    1               5                  10                  15
   GTC TTG CTG ACC ATG GCC TCT GGA AGC CCT CCG ACC CAG CCC TCG CCG      96
   Val Leu Leu Thr Met Ala Ser Gly Ser Pro Pro Thr Gln Pro Ser Pro
               20                  25                  30
   GCC TCG GAT TCC GGC TCT GGC TAC GTT CCG GGC TCG GTC TCT GCA GCC     144
   Ala Ser Asp Ser Gly Ser Gly Tyr Val Pro Gly Ser Val Ser Ala Ala
           35                  40                  45
   TTT GTT ACT TGC CCC AAC GAG AAG GTC GCC AAG GAG ATC GCC AGG GCC     192
   Phe Val Thr Cys Pro Asn Glu Lys Val Ala Lys Glu Ile Ala Arg Ala
       50                  55                  60
   GTG GTG GAG AAG CGC CTA GCA GCC TGC GTC AAC CTC ATC CCT CAG ATT     240
   Val Val Glu Lys Arg Leu Ala Ala Cys Val Asn Leu Ile Pro Gln Ile
   65                  70                  75                  80
   ACA TCC ATC TAT GAG TGG AAA GGG AAG ATC GAG GAA GAC AGT GAG GTG     288
   Thr Ser Ile Tyr Glu Trp Lys Gly Lys Ile Glu Glu Asp Ser Glu Val
                   85                  90                  95
   CTG ATG ATG ATT AAA ACC CAA AGT TCC TTG GTC CCA GCT TTG ACA GAT     336
   Leu Met Met Ile Lys Thr Gln Ser Ser Leu Val Pro Ala Leu Thr Asp
               100                 105                 110
   TTT GTT CGT TCT GTG CAC CCT TAC GAA GTG GCC GAG GTA ATT GCA TTG     384
   Phe Val Arg Ser Val His Pro Tyr Glu Val Ala Glu Val Ile Ala Leu
           115                 120                 125
   CCT GTG GAA CAG GGG AAC TTT CCG TAC CTG CAG TGG GTG CGC CAG GTC     432
   Pro Val Glu Gln Gly Asn Phe Pro Tyr Leu Gln Trp Val Arg Gln Val
       130                 135                 140
   ACA GAG TCA GTT TCT GAC TCT ATC ACA GTC CTG CCA                     468
   Thr Glu Ser Val Ser Asp Ser Ile Thr Val Leu Pro
   145                 150                 155

### (2) INFORMATION FOR SEQ ID NO: 4:

(i) SEQUENCE CHARACTERISTICS:
   (A) LENGTH: 156 amino acids
   (B) TYPE: amino acid
   (D) TOPOLOGY: linear
(ii) MOLECULE TYPE: protein
(xi) SEQUENCE DESCRIPTION: SEQ ID NO: 4:
   Met Pro Ala Leu Leu Pro Val Ala Ser Arg Leu Leu Leu Leu Pro Arg
    1               5                  10                  15
   Val Leu Leu Thr Met Ala Ser Gly Ser Pro Pro Thr Gln Pro Ser Pro
               20                  25                  30
   Ala Ser Asp Ser Gly Ser Gly Tyr Val Pro Gly Ser Val Ser Ala Ala
           35                  40                  45
   Phe Val Thr Cys Pro Asn Glu Lys Val Ala Lys Glu Ile Ala Arg Ala
       50                  55                  60
   Val Val Glu Lys Arg Leu Ala Ala Cys Val Asn Leu Ile Pro Gln Ile
   65                  70                  75                  80
   Thr Ser Ile Tyr Glu Trp Lys Gly Lys Ile Glu Glu Asp Ser Glu Val
                   85                  90                  95
   Leu Met Met Ile Lys Thr Gln Ser Ser Leu Val Pro Ala Leu Thr Asp
               100                 105                 110
   Phe Val Arg Ser Val His Pro Tyr Glu Val Ala Glu Val Ile Ala Leu
           115                 120                 125
   Pro Val Glu Gln Gly Asn Phe Pro Tyr Leu Gln Trp Val Arg Gln Val
       130                 135                 140
   Thr Glu Ser Val Ser Asp Ser Ile Thr Val Leu Pro
   145                 150                 155

### (2) INFORMATION FOR SEQ ID NO: 5:

(i) SEQUENCE CHARACTERISTICS:
   (A) LENGTH: 660 base pairs
   (B) TYPE: nucleic acid
   (C) STRANDEDNESS: double
   (D) TOPOLOGY: linear
(ii) MOLECULE TYPE: cDNA
(ix) FEATURE:
   (A) NAME/KEY: CDS
   (B) LOCATION: 67..537
(xi) SEQUENCE DESCRIPTION: SEQ ID NO: 5:
   TGGTGCGCGC TGTTTTCTAA TCACGTGGCT GCCACCCAGG CCTCTCTGCT CCTGTCTTTT      60
   GTTTGG ATG CCG GCG CTG CTG CCT GTG GCC TCC CGC CTT TTG TTG CTA        108
          Met Pro Ala Leu Leu Pro Val Ala Ser Arg Leu Leu Leu Leu
           1               5                  10
   CCC CGA GTC TTG CTG ACC ATG GCC TCT GGA AGC CCT CCG ACC CAG CCC       156
   Pro Arg Val Leu Leu Thr Met Ala Ser Gly Ser Pro Pro Thr Gln Pro
    15                  20                  25                  30
   TCG CCG GCC TCG GAT TCC GGC TCT GGC TAC GTT CCG GGC TCG GTC TCT       204
   Ser Pro Ala Ser Asp Ser Gly Ser Gly Tyr Val Pro Gly Ser Val Ser
                    35                  40                  45
   GCA GCC TTT GTT ACT TGC CCC AAC GAG AAG GTC GCC AAG GAG ATC GCC       252
   Ala Ala Phe Val Thr Cys Pro Asn Glu Lys Val Ala Lys Glu Ile Ala
                50                  55                  60
   AGG GCC GTG GTG GAG AAG CGC CTA GCA GCC TGC GTC AAC CTC ATC CCT       300
   Arg Ala Val Val Glu Lys Arg Leu Ala Ala Cys Val Asn Leu Ile Pro
            65                  70                  75
   CAG ATT ACA TCC ATC TAT GAG TGG AAA GGG AAG ATC GAG GAA GAC AGT       348
   Gln Ile Thr Ser Ile Tyr Glu Trp Lys Gly Lys Ile Glu Glu Asp Ser
        80                  85                  90
   GAG GTG CTG ATG ATG ATT AAA ACC CAA AGT TCC TTG GTC CCA GCT TTG       396
   Glu Val Leu Met Met Ile Lys Thr Gln Ser Ser Leu Val Pro Ala Leu
    95                 100                 105                 110
   ACA GAT TTT GTT CGT TCT GTG CAC CCT TAC GAA GTG GCC GAG GTA ATT       444
   Thr Asp Phe Val Arg Ser Val His Pro Tyr Glu Val Ala Glu Val Ile
                   115                 120                 125
   GCA TTG CCT GTG GAA CAG GGG AAC TTT CCG TAC CTG CAG TGG GTG CGC       492
   Ala Leu Pro Val Glu Gln Gly Asn Phe Pro Tyr Leu Gln Trp Val Arg
               130                 135                 140
   CAG GTC ACA GAG TCA GTT TCT GAC TCT ATC ACA GTC CTG CCA TGATGAGCCC    544
   Gln Val Thr Glu Ser Val Ser Asp Ser Ile Thr Val Leu Pro
           145                 150                 155
   TGTTCCTGCT CATCATGAAG ATCCCCGCGA TACTTCAACG CCTTCTGACT TCCAGGTGAT     604
   GACTGGGCCC CCAATAAATC CCGTCTTTGG GTCAAAAAAA AAAAAAAAAA AAAAAA         660

### (2) INFORMATION FOR SEQ ID NO: 6:

(i) SEQUENCE CHARACTERISTICS:
   (A) LENGTH: 156 amino acids
   (B) TYPE: amino acid
   (D) TOPOLOGY: linear
(ii) MOLECULE TYPE: protein
(xi) SEQUENCE DESCRIPTION: SEQ ID NO: 6:
   Met Pro Ala Leu Leu Pro Val Ala Ser Arg Leu Leu Leu Leu Pro Arg
    1               5                  10                  15
   Val Leu Leu Thr Met Ala Ser Gly Ser Pro Pro Thr Gln Pro Ser Pro
               20                  25                  30
   Ala Ser Asp Ser Gly Ser Gly Tyr Val Pro Gly Ser Val Ser Ala Ala
           35                  40                  45
   Phe Val Thr Cys Pro Asn Glu Lys Val Ala Lys Glu Ile Ala Arg Ala
       50                  55                  60
   Val Val Glu Lys Arg Leu Ala Ala Cys Val Asn Leu Ile Pro Gln Ile
   65                  70                  75                  80
   Thr Ser Ile Tyr Glu Trp Lys Gly Lys Ile Glu Glu Asp Ser Glu Val
                   85                  90                  95
   Leu Met Met Ile Lys Thr Gln Ser Ser Leu Val Pro Ala Leu Thr Asp
               100                 105                 110
   Phe Val Arg Ser Val His Pro Tyr Glu Val Ala Glu Val Ile Ala Leu
           115                 120                 125
   Pro Val Glu Gln Gly Asn Phe Pro Tyr Leu Gln Trp Val Arg Gln Val
               130                 135                 140
   Thr Glu Ser Val Ser Asp Ser Ile Thr Val Leu Pro
   145                 150                 155

### (2) INFORMATION FOR SEQ ID NO: 7:

(i) SEQUENCE CHARACTERISTICS:
   (A) LENGTH: 45 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: misc_feature
   (B) LOCATION: 1..45
   (D) OTHER INFORMATION: /note= "This is a polylinker and has a 4-bp single stranded overhang at each end. Double stranded only from 5-41"
(xi) SEQUENCE DESCRIPTION: SEQ ID NO: 7:
   AGCTCGTCGA CAGATCTGGT ACCCCGGCGG CCGCACTAGT CTGCA 45

### (2) INFORMATION FOR SEQ ID NO: 8:

(i) SEQUENCE CHARACTERISTICS:
   (A) LENGTH: 60 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: misc_feature
   (B) LOCATION: 1..60
   (D) OTHER INFORMATION: /note= "This is a polylinker and has a 4-bp single stranded overhang at each end. Double stranded only from 5-56"
(xi) SEQUENCE DESCRIPTION: SEQ ID NO: 8:
   TGCAGGAGCT CGTCGACAGA TCTGAATTCC ATATGCCCGG GGCGGCCGCA CTAGTGGTAC        60

### (2) INFORMATION FOR SEQ ID NO: 9:

(i) SEQUENCE CHARACTERISTICS:
   (A) LENGTH: 42 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO: 9:
   TAGAGAGTCG ACCACGTGGC TGCCACCCAG GCCTCTCTGC TC                           42

### (2) INFORMATION FOR SEQ ID NO: 10:

(i) SEQUENCE CHARACTERISTICS:
   (A) LENGTH: 63 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO: 10:
   AAGAGAACTA GTTCAATGAT GATGATGATG ATGTGGCAGG ACTGTGATAG AGTCAGAAAC        60
   TGA                                                                      63